# EUROPÄISCHE PATENTSCHRIFT

(11) **EP 1 648 302 B1**
(45) Veröffentlichungstag und Bekanntmachung des Hinweises auf die Patenterteilung: **24.01.2007**
(21) Anmeldenummer: 03816627.8
(22) Anmeldetag: 24.07.2003
(51) Int. Cl.: A61B 5/103, A61B 5/00

(54) **VORRICHTUNG UND VERFAHREN ZUR MESSUNG SENSIBLER STÖRUNGEN UNTER BERÜCKSICHTIGUNG VERSCHIEDENER UMWELTBEDINGUNGEN**
DEVICE AND METHOD FOR THE MEASUREMENT OF SENSORY DISORDERS WITH REGARD TO VARIOUS ENVIRONMENTAL CONDITIONS
DISPOSITIF ET PROCEDE DE MESURE DE TROUBLES SENSORIELS EN TENANT COMPTE DE DIFFERENTS ETATS DE L'ENVIRONNEMENT

(43) Veröffentlichungstag der Anmeldung: 26.04.2006
(73) Patentinhaber: Schweers, Michael, 40670 Meerbusch (DE); Meyer, Bernd, 40670 Meerbusch (DE); Ziegler, Dan, 40593 Düsseldorf (DE)
(72) Erfinder: Schweers, Michael, 40670 Meerbusch (DE); Meyer, Bernd, 40670 Meerbusch (DE); Ziegler, Dan, 40593 Düsseldorf (DE)
(74) Vertreter: DR. STARK & PARTNER
(86) Internationale Anmeldenummer: PCT/EP2003/008147
(87) Internationale Veröffentlichungsnummer: WO 2005/018452

(56) Entgegenhaltungen:
- EP-A- 0 019 444
- EP-A- 0 569 920
- DE-U- 9 204 961
- US-A- 3 942 515
- US-A- 5 941 833

## Beschreibung

Die Erfindung betrifft ein Verfahren und eine Vorrichtung zur Messung und/oder Feststellung sensibler, d.h neurologischer Störungen, insbesondere bei Neuropathien.

Zur Messung und Feststellung neurologischer Störungen und deren Intensität insbesondere im Einsatzbereich neuropatischer Störungen bei diabetischen Patienten stehen heute verschiedene Instrumente zur Verfügung. Das Spektrum dieser Instrumente ist groß, da sich die Ausprägungen dieser Störungen vielfältig zeigen. Das Krankheitsbild reicht von verminderter Sensibilität bis zu schweren Störungen der kurzen wie langen Nervenfasern. Das Ausmaß der Störung kann als Maß der Intensität angenommen werden.

In Wesentlichen sind folgende Messungen verfügbar:
1. Qualitative Messung
   a. Reflexhammer: Test der verschiedenen Reflexbögen und deren Erfolg. Ein einfacher und bekannter Test, der auf motorischen Steuerungen des menschlichen Körpers basiert. Die entsprechende Stelle (beispielsweise direkt unterhalb der Kniescheibe) wird mit dem Hammer erregt, woraufhin beim gesunden Menschen eine Streckung des Beines initiiert wird. Hierbei ist nicht die Stärke des Ausschlags, sondern die Zeit von Erregung bis Streckung ausschlaggebend.
   b. Diskriminatoren wie z.B. Nadeln ermöglichen einen Test zur Unterscheidung von spitz und stumpf also zur Unterscheidung verschieden großer Flächen auf der Haut. Der Patient ist aufgefordert die gespürte Berührung mitzuteilen.
   c. Monopilament (Haar oder Borste) zum Anregen der Rezeptoren (kitzeln). Mit verschieden vielen, verschieden steifen und verschieden dicken Haare oder Borsten wird die Haut der Patienten erregt. Ohne Sichtkontakt muß der Patient mitteilen ob er diese jeweilige Erregung spürt.
   d. Tiptherm zum Erfühlen von warm/kalt. Hier wird die Eigenschaft verschiedener Materialien Wärme besser zu leiten und abzugeben ausgenutzt. Auf einem Ende eines Kunststoffstabes ist ein gleich großes metallisches Ende befestigt. Abwechselnd und wahllos berührt der Prüfer die Haut des Patienten mit dem Kunststoff- und Metallende.. Der Patient gibt nun die gespürte Erregung (warm/kalt) an.
2. Semi-Qualitative Messung
   Die Stimmgabel zum Erfühlen von Schwingungen (Vibration). Nach Anregung der Stimmgabel wird diese auf bestimmte Stellen der Handwurzel bzw. Fußwurzel gesetzt. Der Patient ist aufgefordert mitzuteilen, wann der die Vibration der abnehmenden Schwingungsamplitude nicht mehr wahrnimmt. Die Übernahme der auf der Stitnmgabeiskala mechanisch/optisch anzeigten Amplitude sollte möglichst zeitnah erfolgen.
3. Quantitative Messung
   a. Thermotest. Mittels eines Neurothersiometers wird die Temperaturempfindung des Patienten festgestellt. Hierzu wird ein Geber entweder Thenar (an der Handwurzel) oder am Fußrücken angesetzt. Allmählich wird nun die Temperatur des Gebers erhöht, bis der Patient den Geber als warm empfindet. In gleicher Weise meldet der Patient den Zeitpunkt, ab dem er die Initiierung bei Abkühlung des Gebers nicht mehr verspürt. Dieser Versuch wird anschließen nochmals wiederholt und das Messergebnis gemittelt. Das Verfahren ist validiert und liefert gute Aussagen über die Intensität bzw. den Fortschritt der vorliegenden Störung.
   b. Vibratest. Wie beim Stimmgabeltest erfolgt hierbei eine Prüfung durch Vibrationsintensität. Die Schwingung wird mechanisch erzeugt und elektronisch gesteuert. Hierdurch können verschiedene Intensitäten wiederholbar eingestellt werden und es kann eine genaue Ablesung erfolgen. Wie beim Thermotest wird mehrfach die Hysterese der Intensität zwischen Auftreten und Verschinden gemessen um die Vibrationsschwelle zu ermitteln.
   c. Elektromyograph Gleichstrom zur Messung der Nervenleitgeschwindigkeit. Mittels eines Erregers wird gewisser Strom gezielt an einzelne Nervenfasern angelegt und mit einer Prüfspitze abgenommen. Die dabei verstrichene Zeit wird gemessen. Ein objektives jedoch sehr aufwendigen Verfahren, welches genaue Messergebnisse liefert.
   d. Elektromyograph Wechselstrom, Neurometa zur Reizstrommessung entspricht in den Grundzügen der Messung mit dem Elektromyographen Gleichstrom. Im Unterschied hierzu jedoch handelt es sich um Wechselstrom, der auf verschiedenen Frequenzen eingestellt werden kann. Dies ermöglicht die Separation bestimmter Nervenfasern und damit eine genauere Aussage gezielt zu großen und kleinen Nervenfasern.

Ein zunehmend wichtiger Bestandteil des heutigen Gesundheitswesens ist die Frühdiagnose sowie die Fähigkeit zu qualitativer und quantitativer Messung bei geringsten Kosten.

Dort, wo Messungen in genügender Zahl präventiv, sowie zur fortlaufenden Beobachtung notwendig sind, fehlt die notwendige Technik. Es ist mehr als verständlich, dass nicht jeder Hausarzt über einen Elektromyographen verfügt, es kann sogar davon ausgegangen werden, das nahezu kein Hausarzt eine solche Investition tätigt. Bei Instituten und Krankenhäusern sieht dies nicht wesentlich anders aus.

Der praktische Hausarzt ist daher gehalten mit den ihm zur Verfügung stehenden Mitteln eine Aussage darüber zu treffen ob bei einem Patienten neuronale z.B. neuropatische Störungen vorliegen und ob sich der Zustand des Patienten verändert hat. So müsste beispielsweise bei Diabetespatienten regelmäßig eine Langzeitkontrolle der neuropatischen Störungen erfolgen. Ohne die heute zur Verfügung stehende aufwendige und teure Technik ist dies nahezu unmöglich. Der Hausarzt greift hier auf qualitative und semi-qualitative Messungen zurück, die ihm lediglich Aussagen im Bereich Ja/Nein geben. An eine quantitative Messung ist hier nicht zu denken. Daher ist es auch nicht verwunderlich, dass die meisten Verfahren nicht validiert sind.

Darüber hinaus erregen alle qualitativen sowie die meisten der quantitativen Messungen große und kleine Fasern wie Berührung und Vibration oder Berührung und Temperatur gleichzeitig. Deutliche Fehlerquellen sind zusätzlich Ablesegenauigkeiten, Störeffekte durch Knochenleitung, sowie die Konzentrationsfähigkeit und der Erschöpfungszustand des Patienten.

Insbesondere die quantitativen Messungen zeigen ihre Schwachstelle in der Langwierigkeit des Messvorganges und der damit verbundenen Veränderung der subjektiven Wahrnehmung des Patienten.

Der Stimmgabeltest stellt heute in der Praxis die wirksamste Messmethode zur Feststellung von neuropatischen Störungen großer Nervenfasern dar.

Störungen kleiner Nervenfasern jedoch benötigen immer noch aufwendige und teure Geräte. Dabei sind es insbesondere die kurzen Fasern, die zumeist als erste eine Störung erfahren und so frühzeitig eine Aussage über eine kommende schwere Erkrankung geben könnten.

Die Aufgabe der Erfindung ist es mittels eines einfachen und kostengünstigen Verfahrens und zugehöriger Vorrichtung die Früherkennung sensibler z.B. neuropatischer Störungen zu ermöglichen und dabei gleichzeitige eine qualitative und zeitnahe Kontrolle des Krankheitsbildes schon beim Hausarzt sicherzustellen.

Diese Aufgabe wird durch ein Verfahren nach Anspruch 1 und eine Vorrichtung nach Anspruch 6 gelöst. Bevorzugte Ausführungsformen sind in den jeweiligen Unteransprüchen genannt.

Der Erfindung liegt die Erkenntnis zugrunde, daß ein Lebewesen, z.B. ein Mensch schon bei einer geringen Luftbewegung das Gefühl hat, es sei deutlich kälter als es der tatsächlichen Lufttemperatur entspricht. Dieser zusätzliche Auskühlungseffekt, der durch den Wind verursacht wird, führt zu einer gefühlten Temperatur, die geringer ist als die tatsächliche Temperatur der Luft und von der Erfindung berücksichtigt wird.

Über seinen Wärmehaushalt ist ein Lebewesen, z.B. der Mensch am engsten mit den atmosphärischen Umweltbedingungen verknüpft. Die gesundheitliche Bedeutung dieser Tatsache hängt mit der engen Vernetzung von Thermo- und Kreislaufregulation zusammen. Eine auf den Menschen bezogene Bewertung von Klima lässt sich folglich über die vom Organismus zu erbringende Anpassungsleistung unter gegebenen klimatischen Bedingungen erreichen. Dazu wird entsprechend der VDI-Richtlinie 3787 Blatt 2 "Methoden zur biometeorologischen Bewertung von Klima und Lufthygiene für die Stadt- und Regionalplanung" das Klima-Michel-Modell eingesetzt. Es liefert eine Aussage über das durchschnittliche subjektive Empfinden des Menschen.

Die subjektiv von einem Lebewesen, z.B. dem Menschen gefühlte Temperatur steigt ebenso unter warmen, sonnigen und windschwachen sommerlichen Bedingungen viel schneller als die tatsächliche Lufttemperatur an. Sie kann im Extremfall in Mitteleuropa bis 15 Grad C über der tatsächlichen Lufttemperatur liegen. Bei angenehmen, milden Bedingungen mit schwachem bis mäßigem Wind kann sie aber auch unter die tatsächliche Lufttemperatur absinken, weil z.B. mit raschem Gehen und einem Anpassen der Bekleidung gerechnet wird. Unter kalter, insbesondere windstarker äußerer Umwelt sinkt die gefühlte Temperatur um bis zu 15 Grad C unter die tatsächliche Lufttemperatur ab. Sonne und Windstille können die gefühlte Temperatur aber auch über die Lufttemperatur klettern lassen.

Im Vergleich zu anderen Bewertungsgrößen bildet die gefühlte Temperatur das Wärmeempfinden physiologisch richtig ab. Die Windchill-Temperatur, ein z. B. für die Klassifizierung kalter Bedingungen genutzter Ansatz, ist ein von der Windgeschwindigkeit abhängiges Maß für die Zeit, die ein Viertelliter Wasser in einem Plastikzylinder benötigt, um zu gefrieren; Sonne oder gar Anpassung der Bekleidung kommen in diesem Ansatz nicht vor.

Ähnlich, wenn auch nicht so stark, steht es zur warmen Seite mit dem sogenannten Discomfort-Index. Die gefühlte Temperatur wird nach der VDI-Richtlinie 3787 Blatt 2 (Entwurf) in eine physiologisch gerechte Bewertung des thermischen Empfindens umgesetzt, den sogenannten Predicted Mean Vote (PMV)-Wert. Dieser Wert entspricht dem vorhergesagten Durchschnittswert der thermischen Beurteilung auf einer psycho-physikalischen Skala von -3 = kalt bis +3=heiß.

Das thermische Empfinden basiert auf der Leit- und Funktionsfähigkeit der kurzen Nervenfasern eines Lebewesens, wie z.B. des Menschen. Liegen bei diesen Nervenfasern Störungen, z.B. neuropatische Störungen vor, so verändert sich hierdurch die jeweilige subjektive Wahrnehmung. Entsprechend ist erfindungsgemäß eine veränderte thermische Beurteilung ein Indiz für eine Störung.

Das erfindungsgemäße Verfahren und die Vorrichtung berücksichtigen nicht nur den bereits beschriebenen Efffekt, sondern wenigstens einen zusätzlichen Parameter, der einen Einfluss auf die gefühlte Temperatur hat, wie z.B. wenigstens einen aus Lufttemperatur, Luftfeuchte, Hauttemperatur, Hautfeuchte, etc.

Diese beispielhaft genannten Parameter haben Einfluss auf die gefühlte Temperatur, so dass es erfindungsgemäß vorgesehen ist wenigstens einen der Parameter, bevorzugt mehrere zur Bestimmung der gefühlten Temperatur heranzuziehen.

Beispielsweise ist der Windchill-Effekt von der Luftfeuchte und Temperatur abhängig, d.h. bei gleicher Windgeschwindigkeit und Lufttemperatur ist das subjektive Empfinden der gefühlten Temperatur bei unterschiedlichen Luftfeuchtigkeiten ebenso unterschiedlich, was bei der Vorrichtung und im Verfahren zur Bestimmung der gefühlten Temperatur berücksichtigt wird.

Ebenso kann beispielsweise Verdunstungskälte beim Schwitzen einen Einfluss haben, so dass es bevorzugt vorgesehen ist, die Hautfeuchtigkeit und/oder die Hauttemperatur zu Erfassen und in die Bestimmung der gefühlten Temperatur einfließen zu lassen.

Gemäß der Erfindung ist es dafür vorgesehen, dass die Vorrichtung wenigstens einen internen oder externen Sensor umfasst, mittels dem wenigstens ein Parameter der Umgebeung und/oder des Lebewesens meßbar ist, um einen solchen Parameter, der einen Einfluss auf die gefühlte Temperatur hat, bei der Bestimmung der gefühlten Temperatur einfließen zu lassen. Bevorzugt werden mehrere solcher Sensoren eingesetzt, um mehrere, z.B. die obengenannten Parameter gleichzeitig oder nacheinander messen zu können.

Die erfindungsgemäße Vorrichtung, z.B. ein mobiles Meßgerät kann beliebig klein und handlich aufgebaut und so nahezu überall genutzt werden. Da die Erfindung validierbar ist kann ein Maß für den Grad einer Störung festgelegt werden.

Bevorzugt können Verfahren und Vorrichtung durch Unterstützung einer elektronischen Regelung der Luftgeschwindigkeit bei konstanten Abstand zum Messpunkt bzw. variablen Abstand, bei konstanten Luftstrom einfach und sicher angewendet werden. Je nach Anwendung und Handhabung kann auf Basis der Luftgeschwindigkeit (Drehzahl, Aufbau und Konstruktion des Ventilators, angeschlossene bzw. integrierte Messfühler für einzelne oder verschiedene Umweltbedingungen) sowie des Abstands die Berechnung der gefühlten Temperatur z.B. auf Basis des o.a. Michel-Klima Modells oder eines anderen insbesondere anerkannten Modells erfolgen.

Im einfachsten Fall, in einem geschlossenen Raum, bei durchschnittlicher Raumtemperatur, gemessen auf der unbekleideten Haut kann eine Störung oder deren Grad anhand von Abstand und Luftgeschwindigkeit abgelesen bzw. ermittelt werden, wobei die abgelesenen oder ermittelten Werte einem behandelnden Arzt Hinweise für eine durchzuführende Diagnose geben können.

Die erfindungsgemäße Vorrichtung ist handlich, einfach zu bedienen, einfach anzuwenden, elektronisch gesteuert, sicher auszuwerten, arbeitet berührungslos, schließt Störeinflüsse aus, erlaubt Mehrfachmessung, ist schnell, ist validierbar und kostengünstig.

Aufgrund seiner Beschaffenheit kann die Vorrichtung von jedem Hausarzt erworben und sicher eingesetzt werden.

Die Vorrichtung ermöglicht mit einfachen Mitteln ein Indiz für eine Störung, insbesondere neuropatische Störung oder deren Grad festzustellen, welches einer Diagnose zugrunde gelegt werden kann, frühzeitig eine entsprechende Behandlung einzuleiten und Medikation festzulegen. Eine Früherkennung von z.B. Neuropathien kann leichter vorgenommen und Krankheiten können wirkungsvoll und kostengünstig behandelt werden. Die Vorrichtung bildet eine Anwendung der subjektiv gefühlten Temperatur zur Feststellung sensibler, d.h. nervlicher Störungen.

Ausführungsbeispiele der Erfindung sind in den Zeichungen dargestellt und werden im Folgenden näher beschrieben:

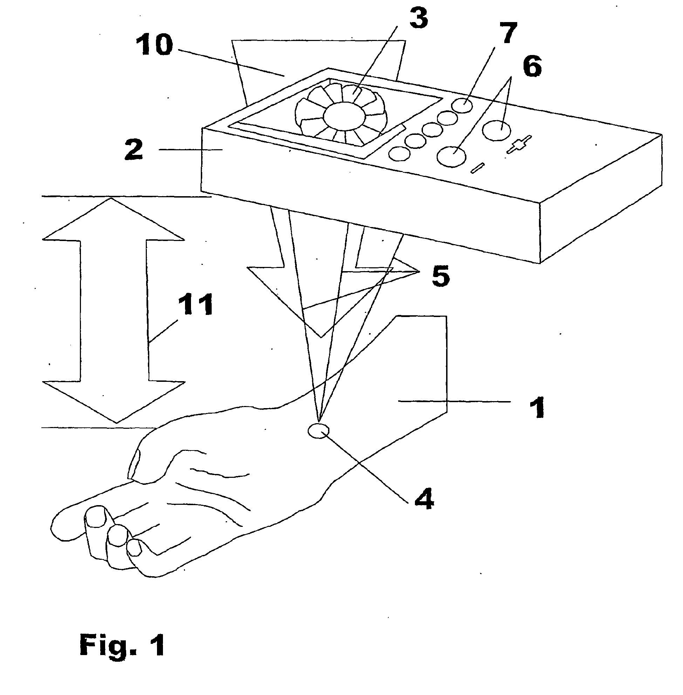

Es zeigen
- Figur 1:: eine perspektivische Ansicht einer thenaren Messung mit festem Abstand und variabler Luftgeschwindigkeit

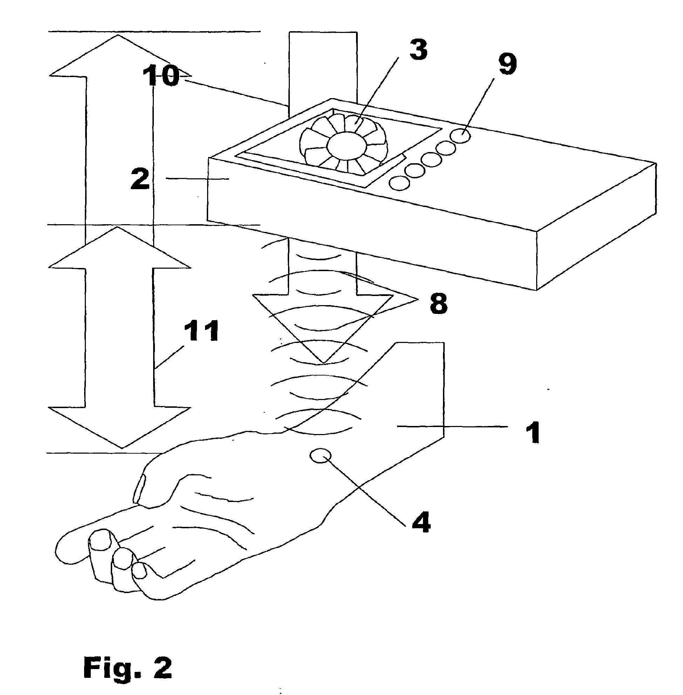

- Figur 2:: eine perspektivische Ansicht einer thenaren Messung mit variablem Abstand und konstanter Luftgeschwindigkeit

Bei einem Patienten (1) erfolgt mittels einer Messvorrichtung (2) eine z.B. thenare (Innenhandwurzel) Messung an der unbekleideten Haut (4). Alternativ kann eine Messung auch an anderen Positionen wie. z.B. Fußwurzel etc. erfolgen.

Der zu messende Körperteil sollte bevorzugt ruhig und geschützt von Zugluft gehalten werden. Hierbei ist bedingt durch die Kürze der Messung eine Auflage nicht zwingend erforderlich. Die Vorrichtung umfasst z.B. einen Ventilator (3) zur Erzeugung eines Luftstroms (10). Bevorzugt wird die Messung ohne Sichtkontakt durchgeführt, um einen Einfluss auf Grund eines akustischen Geräusches des Ventilators zu vermeiden.

Im Falle einer Messung mit konstantem Abstand wird das Messgerät eingeschaltet und der feste Abstand eingestellt. Dies kann insbesondere durch die Verwendung von Laserdioden erfolgen, deren Lichtstrahlen (5) sich in einem vorgegebenen Abstand zum Messgerät (2) schneiden. Auch können die Dioden auf einen gemeinsamen Fokus eingestellt sein.

Insbesondere bei Verwendung von drei Laserdioden die in einem Winkelabstand von 120° angeordnet sind, ist nicht nur ein fester Abstand, sondern auch eine lotrechte Position des Messgerätes über der Messstelle (4) sicher einstellbar.

Anschließend erfolgt eine allmähliche Veränderung (6) eines für die Messung wesentlichen Parameters, der eine Änderung der gefühlten Temperatur bewirkt, z.B. der Luftgeschwindigkeit und/oder Luftfeuchte und/oder Lufttemperatur analog oder in diskreten Stufen, wobei der Abstand konstant gehalten wird, z.B. kann die Luftgeschwindigkeit über die Drehzahl des Ventilator verändert werden.

Anhand der eingestellten Parameter sowie bevorzugt gemessener Parameter (mittels integrierten und/oder externen Messfühler) sowie insbesondere ergänzend auf Basis des zugrundegelegten Klima-Models (z.B. Klima-Michael-Model, Windchill, ...) kann durch das Meßgerät die gefühlte Temperatur berechnet und an einer entsprechenden Anzeige (7) dargestellt.

Die Parameter und damit die gefühlte Temperatur können nun durch den Bediener des Messgeräts geändert werden, bis der Patient subjektiv den Luftzug (die Temperaturveränderung) wahrnimmt. Der angezeigte Wert z.B. der gefühlten Temperatur wird notiert. Der Parameter, z.B. die Drehzahl /Luftfeuchte/Lufttemperatur wird bevorzugt um eine weitere Stufe geändert, z.B. erhöht und anschließend vermindert bis der Patient die Erregung nicht mehr spürt.

Auch dieser Wert der gefühlten Temperatur wird notiert. Das Notieren kann auch geräteintern durch eine Speicherung erfolgen. Um Fehler durch Störeinflusse, wie Anspannung des Patienten oder Geräusche auszuschließen kann diese Hysteresemessung mehrfach wiederholt werden. Die notierten Werte und/oder deren Mittelwerte können nun Aufschluss über den Grad der Störung geben, bzw. einem Arzt Anhaltspunkte für eine Diagnose bereitstellen.

Erfolgt die Messung mittels konstantem Luftstrom (10) wird das Messgerät (2) z.B. eingeschaltet und beginnend mit großer Entfernung dem Messpunkt (4) genähert. Bevorzugt kann mittels einer Abstandsmessung (8), z.B. durch akustische oder optische Signallaufzeitmessung, die Entfernung zum Messpunkt analog oder in diskreten Stufen an einer entsprechenden Anzeige (9) angezeigt werden.

Die Entfernung wird nun vermindert, bis der Patient subjektiv den Luftzug (die Temperaturveränderung) wahrnimmt. Der angezeigte Wert der Entfernung und/oder der gefühlten Tempertur wird notiert. Die Entfernung wird bevorzugt noch weiter verkürzt und anschließend erhöht bis der Patient die Erregung nicht mehr spürt. Auch hier wird der angezeigte Wert notiert und z.B. die Messung zum Ausschluss von Störeinflüssen mehrfach wiederholt. Die notierten Werte sowie deren Mittel können nun Aufschluss über den Grad der Störung geben.

Der Einsatz eines solchen Messgerätes ist insbesondere nicht beschränkt auf Hand- oder Fußwurzelmessung, nicht beschränkt auf den mobilen Einsatz und nicht beschränkt auf den menschlichen Körper.

Durch die Messung von Umweltparametern, wie z.B. der Luftfeuchte und/oder der Lufttemperatur, Parametern des Lebewesens, wie z.B. der Hautfeuchte und/oder der Hauttemperatur oder allgemein wenigstens eines Parameters, der die gefühlte Temperatur beinflusst, kann die gefühlte Temperatur genauer ermittelt werden. Eine Vorrichtung welche integrierte oder externe Sensoren zur Erfassung solcher die gefühlte Temperatur beeinflussender Parameter aufweist, kann erfindungsgemäß eine genauere Aussage über die gefühlte Temperatur geben.

## Patentansprüche

1. Verfahren zur Messung und/oder Feststellung sensibler Störungen insbesondere bei Neuropathien, wobei mittels einer Vorrichtung (2) ein Luftstrom (10) auf einen Messpunkt (4) am Körper eines Lebewesens, insbesondere eines Menschen, geleitet wird, um während eines Messvorgangs das thermische Empfinden des Lebewesens an diesem Messpunkt (4) zu beeinflussen, **dadurch gekennzeichnet, dass** das thermische Empfinden und/oder eine Änderung des thermischen Empfindens mit einer gefühlten Temperatur korreliert wird und zur Bestimmung der gefühlten Temperatur vor und/oder während des Messvorgangs wenigstens ein Parameter der Umgebung und/oder des Lebewesens erfasst und ausgewertet wird.

2. Verfahren nach Anspruch 1, **dadurch gekennzeichnet, dass** die Messung mittels konstantem Luftstrom (10) bei variablem Abstand zwischen Vorrichtung (2) und Messpunkt (4) erfolgt.

3. Verfahren nach Anspruch 1, **dadurch gekennzeichnet, dass** die Messung mittels variablem Luftstrom (10) bei konstantem Abstand zwischen Vorrichtung (2) und Messpunkt (4) erfolgt.

4. Verfahren nach einem der vorherigen Ansprüche, **dadurch gekennzeichnet, dass** der Messpunkt mittels der Vorrichtung optisch bestimmt wird insbesondere durch Überlagerung dreier Lichtstrahlen.

5. Verfahren nach einem der vorherigen Ansprüche, **dadurch gekennzeichnet, dass** in die Bestimmung der gefühlten Temperatur wenigstens einer der Parameter Umgebungs-Lufttemperatur, Luftfeuchte, Hauttemperatur oder Hautfeuchtigkeit eingeht.

6. Vorrichtung zur Messung und/oder Feststellung sensibler Störungen, insbesondere bei Neuropathien, wobei sie Mittel zur Erzeugung eines Luftstromes aufweist, der auf einen Messpunkt am Körper eines Lebewesens richtbar ist, und wenigstens einen internen oder externen Sensor aufweist, mit dem wenigstens ein Parameter der Umgebung und/oder des Lebewesens messbar ist, der zur Bestimmung einer am Messpunkt gefühlten Temperatur heranziehbar ist, und wobei die Vorrichtung Mittel umfasst zur Bestimmung einer gefühlten Temperatur.

7. Vorrichtung nach Anspruch 7, **dadurch gekennzeichnet, dass** mit einem Sensor wenigstens einer der Parameter Lufttemperatur, Luftfeuchte, Hauttemperatur, Hautfeuchte meßbar ist.

8. Vorrichtung nach einem der vorherigen Ansprüche, **dadurch gekennzeichnet, dass** der Luftstrom variabel einstellbar/regelbar ist, insbesondere wobei eine eingestellte Luftstromgeschwindigkeit und/oder eingestellter Volumenstrom in die Bestimmung der gefühlten Temperatur eingeht.

9. Vorrichtung nach einem der vorherigen Ansprüche, **dadurch gekennzeichnet, dass** sie Mittel umfasst zur Bestimmung und/oder Anzeige und/oder Speicherung eine gefühlten Temperatur.

10. Vorrichtung nach einem der vorherigen Ansprüche, **dadurch gekennzeichnet, dass** sie Mittel umfasst zur Einstellung eines gewünschten Abstandes zwischen Vorrichtung und Messpunkt.

11. Vorrichtung nach Anspruch 10, **dadurch gekennzeichnet, dass** die Leuchtmittel, insbesondere Leuchtdioden oder Laserdioden umfassen, deren Lichtstrahlen sich in einem insbesondere vorherbestimmbaren Abstand von der Vorrichtung schneiden.

## Claims

1. A device for measuring and/or establishing sensory disorders especially neuropathies, whereby a device (2) directs an air stream (10) onto a measuring point (4) on the body of a living organism, especially a person to influence the thermal sensitivity and/or change in the thermal sensitivity during a measurement process, **characterized in that** the thermal sensitivity and/or change in the thermal sensitivity is correlated with perceived temperature and for determining the perceived temperature before and/or during a measurement process at least on parameter of the environment and/or living organism is detected and evaluated.

2. The method according to claim 1 **characterized in that** the measurement is effected with a constant air stream (10) and a variable distance between the device (2) and the measurement point (4).

3. The device according to claim 1 **characterized in that** the measurement is effected by means of a variable air stream (10) at a constant distance between the device (2) and the measuring point (4).

4. The method according to one of the preceding claims **characterized in that** the measuring point is determined optically by the device and by the superimposition or three light beams.

5. The method according to one of the preceding claims, **characterized in that** in the determination of the perceived temperature at least one of the parameters: ambient air temperature, air moisture content, skin temperature or skin moisture is used as an input.

6. A device for the measurement and/or determination of sensory disorders, especially neuropathies, whereby the device has means for producing an air stream which is directed against a measuring point on the body of the living organism and has an external or internal sensor with which at least one parameter of the living organism or environment in order to determine a perceived temperature at the measuring point, whereby the device contains means to determine the perceived temperature.

7. The device according to claim 6 **characterized in that** at least one of the parameters: air temperature, air humidity, skin temperature and skin moisture is measurable by a sensor.

8. The device according to one of the preceding claims **characterized in that** the air stream is variably adjustable or controllable and especially such that an air velocity can be set and/or a volume stream can be adjusted in the determination of the perceived temperature.

9. The device according to one of the preceding claims **characterized in that** it includes means for determining and/or indicating and/or storing a perceived temperature.

10. The device according to one of the preceding claims **characterized in that** it include means for adjusting a desired distance between the device and the measured point.

11. The device according to claim 10 **characterized in that** the light sources comprise light emitting diodes or laser diodes whose light beams intersect at a predetermined distance from the device.

## Revendications

1. Un dispositif pour mesurer et/ou établir les troubles sensoriels en particulier les neuropathies, par lequel le dispositif (2) dirige un souffle d'air (10) sur un point à mesurer (4) du corps d'un organisme vivant, spécialement d'une personne afin d'influencer la sensibilité thermique et/ou le changement de la sensibilité thermique durant la mesure en cours, **caractérisé par** la sensibilité thermique et/ou le changement de la sensibilité thermique à se corréler avec la température apparente et pour déterminer la température apparente avant et/ou pendant la mesure en cours au moins sur un paramètre des conditions atmosphériques et/ou de l'organisme vivant à être détecté et évalué.

2. La méthode selon la déclaration 1 **caractérisée en ce que** la mesure soit effectuée avec un souffle constant d'air (10) et une distance variable entre le dispositif (2) et le point à mesurer.

3. Le dispositif selon la déclaration 1 **caractérisé en ce que** la mesure soit effectuée à l'aide d'un souffle d'air variable(10) à une distance constante entre le dispositif (2) et le point à mesurer (4)

4. La méthode selon une des déclarations précédentes **caractérisé en ce que** le point à mesurer soit déterminé optiquement par le dispositif et par la superposition de trois faisceaux lumineux.

5. La méthode selon une des déclarations précédentes **caractérisée en ce que** dans la détermination de la température apparente au moins un des paramètres : la température ambiante, le contenu d'humidité d'air, la température de peau ou son humidité soit entrée comme donnée.

6. Un dispositif pour la mesure et/ou la détermination des troubles sensoriels, particulièrement la neuropathie, par lequel le dispositif ait les moyens de produire un souffle d'air dirigé directement contre le point à mesurer du corps de l'organisme vivant et ait une sonde externe ou interne avec laquelle au moins un paramètre de l'organisme vivant ou de l'environnement soit mesurable de façon a déterminer la température apparente au point à mesurer, d'ou l'appareil a les moyens de déterminer la température apparente.

7. Le dispositif selon la déclaration 6 **caractérisé** à ce qu'un des paramètres : la température de l'air, l'humidité d'air, la température de peau et l'humidité de peau soit mesurable par des sondes.

8. Le dispositif selon une des déclarations précédentes **caractérisé en ce que** le souffle d'air soit variablement réglable ou contrôlable et en particulier à pouvoir régler la vitesse de l'air et/ou ajuster le volume du souffle dans la détermination de la température apparente.

9. Le dispositif selon une des déclarations précédentes **caractérisé en ce qu'**il inclut des moyens pour déterminer et/ou indiquer et/ou stocker la température apparente.

10. Le dispositif selon une des déclarations précédentes **caractérisé en ce qu'**il inclut des moyens pour ajuster une distance désirée entre le dispositif et le point à mesurer.

11. Le dispositif selon la déclaration 10 **caractérisé en ce que** les sources lumineuses comportant des diodes électroluminescentes ou des diodes de laser dont les faisceaux lumineux s'entrecroisent à une distance prédéterminée du dispositif.
